# EUROPEAN PATENT APPLICATION

(11) **EP 4 462 114 A1**
(43) Date of publication of application: **13.11.2024**
(21) Application number: 23737289.1
(22) Date of filing: 05.01.2023
(51) Int. Cl.: G01N 27/62

(54) **METHOD AND KIT FOR IDENTIFYING SUGAR-BOUND AMINO ACID SITE IN PROTEIN**

(30) Priority: 06.01.2022 JP 2022001248
(71) Applicant: FUJIFILM Corporation, Tokyo 106-8620 (JP)
(72) Inventor: MATSUDA Shun, Ashigarakami-gun, Kanagawa 258-8577 (JP)
(74) Representative: Hoffmann Eitle
(86) International application number: PCT/JP2023/000038
(87) International publication number: WO 2023/132338

(57) **Abstract**

An object of the present invention is to provide a method of identifying an amino acid site in a protein, to which a sugar is bound more accurately than in the related art, and a kit for performing the method. According to the present invention, there is provided a method for identifying an amino acid site in a protein, to which a sugar is bonded, the method including a first mass spectrometry step of subjecting a fragmented protein to mass spectrometry; for a protein in which the bonding of the sugar has been confirmed in the first mass spectrometry step, a substitution-modification step of substituting and/or modifying an amino acid of the protein at the amino acid site in the protein, to which the sugar is bonded and a fragmentation step of fragmenting the protein; and a second mass spectrometry step of subjecting the substituted and/or modified, and fragmented protein obtained by the substitution-modification step and the fragmentation step, to mass spectrometry.

## Description

### Technical Field

The present invention relates to a method for identifying an amino acid site in a protein, to which a sugar is bonded. The present invention further relates to a kit for performing the method for identifying an amino acid site.

### Background Art

The modification of a protein with a sugar chain is one of important post-translational modifications that regulate the function of the protein. In biopharmaceuticals such as antibodies, it has been found that modification of a protein with a sugar chain may affect a half-life in blood and a biological activity, and it is necessary to control the modification of a protein with a sugar chain for quality control. In order to control the modification of a protein with a sugar chain, it is necessary to identify an amino acid site in the protein, to which a sugar is bonded.

Patent Document 1 describes a method for identifying a bonding region of a sugar chain in a glycoprotein, the method including a step of fluorescently labeling a sugar chain in the glycoprotein, a step of acquiring a glycopeptide fragment by fragmenting the glycoprotein, a step of acquiring fluorescence analysis data by fluorescence detection using liquid chromatography, a step of acquiring mass spectrometry data by mass detection using a mass spectrometer, and a step of extracting a peak of a mass-to-charge ratio (m/z) corresponding to a peak of a fluorescence intensity by comparing the fluorescence analysis data with the mass spectrometry data.

### Prior Art Documents

### Patent Documents

Patent Document 1: JP2019-52995A

### SUMMARY OF THE INVENTION

### Object to be solved by the invention

In the method described in Patent Document 1, in a case where a plurality of serine or threonine residues are present in a protein (peptide) in which the GlcNAc-modification is detected, the GlcNAc-modified amino acid residue could not be specified.

An object of the present invention is to provide a method of identifying an amino acid site to in a protein, which a sugar is bound. Another object of the present invention is to provide a kit for performing the method for identifying an amino acid site in a protein, to which a sugar is bonded.

### Means for solving the object

As a result of intensive studies to achieve the above-described object, the present inventors have found that, by performing a first mass spectrometry step of subjecting a fragmented protein to mass spectrometry, then, for a protein in which the bonding of the sugar has been confirmed in the first mass spectrometry step, performing a substitution-modification step of substituting and/or modifying an amino acid at an amino acid site in the protein, to which a sugar is bonded, a fragmentation step of fragmenting the protein, and a second mass spectrometry step of subjecting the obtained protein to mass spectrometry, a modificated site with GlcNAc in the virus capsid modified with the O-type sugar chain can be identified. The present invention has been completed based on the above findings.

That is, according to an aspect of the present invention, the following invention is provided.
<1> A method for identifying an amino acid site in a protein, to which a sugar is bonded, the method comprising: a first mass spectrometry step of subjecting a fragmented protein to mass spectrometry; for the protein in which the bonding of the sugar has been confirmed in the first mass spectrometry step, a substitution-modification step of substituting and/or modifying an amino acid of the protein at the amino acid site in the protein, to which the sugar is bonded and a fragmentation step of fragmenting the protein; and a second mass spectrometry step of subjecting the substituted and/or modified, and fragmented protein obtained by the substitution-modification step and the fragmentation step, to mass spectrometry.
<2> The method according to <!>, further comprising comparing a mass spectrometry result obtained in the first mass spectrometry step with a mass spectrometry result obtained in the second mass spectrometry step.
<3> The method according to <1> or <2>, in which the amino acid to which the sugar is bonded is serine and/or threonine.
<4> The method according to any one of <1> to <3>, in which the substitution-modification step is a substitution reaction by a Michael addition reaction to an amino acid site from which the sugar has been eliminated by a β-elimination reaction.
<5> The method according to any one of <1> to <4>, in which the Michael addition reaction in the substitution-modification step is a reaction with a cyclic active methylene compound.
<6> The method according to any one of <1> to <5>, in which the Michael addition reaction in the substitution-modification step is a reaction with a pyrazolone compound, a barbituric acid compound, a dimedone compound, or a hydroxycoumarin compound.
<7> The method according to any one of <1> to <6>, in which the Michael addition reaction in the substitution-modification step is a reaction with a pyrazolone compound.
<8> The method according to <7>, in which the pyrazolone compound is 3-methyl-1-phenyl-pyrazolone.
<9> The method according to any one of <1> to <8>, in which the sugar is N-acetylglucosamine.
<10> The method according to any one of <1> to <9>, in which the protein is a capsid of a virus.
<11> The method according to <10>, in which the virus is an adeno-associated virus.
<12> The method according to <11>, in which the virus is an adeno-associated virus 5 referred to as AAV5.
<13> The method according to any one of <1> to <12>, in which an amount of the protein to be subjected to the spectrometry is 100 fmol or less.
<14> A kit for performing the method according to any one of <1> to <13>, the kit comprising a cyclic active methylene compound.
<15> The kit according to <14>, further comprising a unit for purifying a fragmented protein.

### Effect of the invention

According to the method and the kit of the present invention, the amino acid site in the protein, to which the sugar is bound can be identified more accurately than in the related art.

### BRIEF DESCRIPTION OF THE DRAWINGS

Fig. 1 shows an MS/MS spectrum of an O-GlcNAc-bonded peptide detected from a fragmented AAV capsid.
Fig. 2 shows an MS/MS spectrum of an O-GlcNAc-bonded peptide detected from a fragmented AAV capsid.
Fig. 3 shows an MS/MS spectrum of a PMP-modified and fragmented, AAV capsid-derived peptide.
Fig. 4 shows an MS/MS spectrum of a PMP-modified and fragmented, AAV capsid-derived peptide.
Fig. 5 shows an extracted chromatogram of a precursor ion for an unmodified peptide preparation and an internal standard (IS).
Fig. 6 shows an extracted chromatogram of a precursor ion for a peptide preparation in which S469 is modified and the internal standard (IS).
Fig. 7 shows a calibration curve.

### DESCRIPTION OF THE PREFERRED EMBODIMENTS

Hereinafter, the contents of the present invention will be described in detail. In the present specification, a numerical range indicated using "to" means a range including numerical values described before and after "to" as a minimum value and a maximum value, respectively. In addition, in the present specification, the peptide and the protein have the same meaning and are interchangeable with each other.

AAV means adeno-associated virus. The number immediately after AAV, such as AAV2, AAV5, or AAV9, represents the serotype of AAV.

LC means liquid chromatography.

MS means a mass spectrum.

ESI means electrospray ionization.

HCD means higher-energy collisional dissociation.

A method for identifying an amino acid site in a protein, to which a sugar is bonded according to the present invention includes a first mass spectrometry step of subjecting a fragmented protein to mass spectrometry; for a protein in which the bonding of the sugar has been confirmed in the first mass spectrometry step, a substitution-modification step of substituting and/or modifying an amino acid of the protein at the amino acid site in the protein, to which the sugar is bonded and a fragmentation step of fragmenting the protein; and a second mass spectrometry step of subjecting the substituted and/or modified, and fragmented protein obtained by the substitution-modification step and the fragmentation step, to mass spectrometry.

In the first mass spectrometry step, the glycosylated protein can be identified. According to the method of the embodiment of the present invention, since it is possible to specify the labeled amino acid residue (that is, the glycosylated amino acid residue) in the range of the amino acid residue which has been found to glycosylated in the first mass spectrometry step, by the subsequent substitution-modification step, the fragmentation step, and the second mass spectrometry step, the glycosylation site can be specified with high accuracy. In the present invention, the glycosylation site can be specified by comparing the mass spectrometry result obtained in the first mass spectrometry step with the mass spectrometry result obtained in the second mass spectrometry step.

In the present invention, the identification of the amino acid site to which the sugar is bonded means the determination of the position of the amino acid to which the sugar is bonded.

The protein is not particularly limited as long as it is a protein that may be glycosylated. The protein may be any of a naturally derived protein, a protein obtained by a gene recombination method, or a chemically synthesized protein. Examples of the protein obtained by a gene recombination method include a recombinant protein expressed in a host cell.

The cell as a host is preferably a eukaryotic cell and more preferably a mammalian cell. Examples of the mammalian cell include a human cell, a mouse cell, a rat cell, a monkey cell, and a hamster cell, but the cells are not particularly limited. The human cell can be preferably used. Examples of the cell include mouse myeloma (NSO) cell lines, Chinese hamster ovary (CHO) cell lines, HT1080, H9, HepG2, MCF7, MDBK Jurkat, NIH3T3, PC12, a baby hamster kidney (BHK) cell, VERO, SP2/0, YB2/0, Y0, C127, an L cell, COS (for example, COS1 and COS7), QC1-3, a human embryo-derived kidney (HEK293) cell, VERO, PER. C6, HeLa, EB1, EB2, EB3, and oncolytic or hybridoma cell lines. The cell is preferably a HEK293 cell or a CHO cell, and more preferably a HEK293 cell.

The protein is preferably a virus capsid. The virus capsid, also referred to as a capsid, is a protein shell surrounding a virus genome.

Examples of the virus include an adeno-associated virus, an adenovirus, a retrovirus, a lentivirus, and a Sendai virus, but the virus is not particularly limited. The virus is preferably an adeno-associated virus, and for example, may be any of AAV1, AAV2, AAV3, AAV4, AAV5, AAV6, AAV7, AAV8, AAV9, AAV10, or AAV11, and more preferably AAV5, AAV8, or AAV9, and still more preferably AAV5.

Since AAV is produced in a host cell, it is considered that the capsid is glycosylated by an enzyme derived from the host cell, which may affect the drug efficacy or the side effect of AAV. In the guidance of the Food and Drug Administration (FDA) published in January 2020, it is recommended to analyze characteristics of the therapeutic AAV, such as a glycosylation site, in addition to the molecular weight and the size. However, there is almost no knowledge about the glycosylation of AAV, and the situation is unclear even whether or not AAV is glycosylated, and an analysis method has not been established. In one embodiment of the present invention, the GlcNAc-modified site of the AAV can be analyzed by the method according to the embodiment of the present invention. As a result, the method according to the embodiment of the present invention can be used for quality evaluation of AAV

The protein may be a protein other than the virus capsid, and may be for example, an antibody (for example, a human antibody, a humanized antibody, a chimeric antibody, a mouse antibody, a bispecific antibody, or the like), a fragmented immunoglobulin, a single-chain antibody (scFv), or the like. Examples of the fragmented immunoglobulin include Fab, F(ab')₂, and Fv. The class of the antibody is also not particularly limited, and it may be any class of IgG such as IgG1, IgG2, IgG3, or IgG4, IgA, IgD, IgE, or IgM. However, IgG or IgM is preferable in a case of being used as a medicine. The amino acid residue constituting the protein is not particularly limited, but is preferably an amino acid residue including serine or threonine.

In the present invention, the amount of the protein to be subjected to the spectrometry may be 1,000 fmol or less, 500 fmol or less, 200 fmol or less, or 100 fmol or less.

The sugar may be any of a monosaccharide, a disaccharide, a trisaccharide, a tetrasaccharide, an oligosaccharide (a saccharide in which about 2 to about 20 molecules of saccharides are bonded), or a polysaccharide (a saccharide in which a large number of monosaccharides are polymerized), but is preferably a monosaccharide. Among saccharides, the monosaccharide is a general term for a sugar which cannot be further hydrolyzed. Specific examples of the monosaccharide include N-acetylglucosamine (referred to as GlcNAc) having a structure shown below, but the monosaccharide is not particularly limited, and N-acetylglucosamine is preferable.

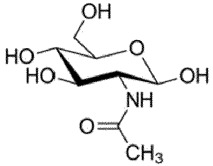

The amino acid to which the sugar is bonded is not particularly limited, but is, for example, serine, threonine, or asparagine, and is preferably serine and/or threonine.

In the present invention, the modification rate of the sugar (for example, GlcNAc) in the protein (for example, virus capsid) represents a ratio of the protein which has been modified among the protein molecules contained in the sample, and means (the number of molecules of the protein which has been modified)/(the number of protein molecules contained in the sample). The modification rate of the sugar in the protein may be, for example, less than 1%, preferably 0.8% or less, 0.6% or less, 0.5% or less, or 0.4% or less. The quantification of the modification rate of sugar in the protein can be performed as described in Examples below.

In the analysis of a protein, the smaller the amount of the protein is, the more sensitive analysis is required, and the lower the modification rate is, the more sensitive analysis is required. According to the present invention, in a protein having a modification rate of a sugar of 0.0001% or more and having an amount of less than 1% and 1 fmol or more and 1,000 fmol or less, an amino acid site to which the sugar chain is bonded can be identified. In addition, in a protein having a modification rate of a sugar of 0.001% or more and 0.6% or less and having an amount of 5 fmol or more and 500 fmol or less, an amino acid site to which the sugar chain is bonded can be identified. Furthermore, in a protein having a modification rate of a sugar of 0.005% or more and 0.4% or less and having an amount of 10 fmol or more and 100 fmol or less, an amino acid site to which the sugar chain is bonded can be identified.

### <For substitution-modification step>

In the substitution-modification step, the amino acid of the amino acid site in the protein, to which the sugar is bonded is substituted and/or modified for the protein in which the bonding of the sugar has been confirmed in the first mass spectrometry step.

The substitution-modification step is preferably a Michael addition reaction to an amino acid site from which a sugar has been eliminated by a β-elimination reaction. In the present disclosure, the β-elimination reaction and the Michael addition reaction can be performed in the same step, and in this case, a reaction occurs in which a substituent is replaced on the same atom of the compound, apparently.

The Michael addition reaction in the substitution-modification step is preferably a reaction with a cyclic active methylene compound, more preferably a reaction with a pyrazolone compound, a barbituric acid compound, a dimedone compound, or a hydroxycoumarin compound, and particularly preferably a reaction with a pyrazolone compound.

The pyrazolone compound is not particularly limited as long as it is a compound having a pyrazolone group, and examples thereof include 3-methyl-1-phenyl-5-pyrazolone (PMP), 1,3-dimethyl-pyrazolone (DP), 3-methyl-1-p-tolyl-5-pyrazolone (MTP), and 3-methyl-1-(quinolin-8-yl)-1H-pyrazole-5(4H)-one. The pyrazolone compound may be used in combination of two or more types of compounds. The pyrazolone compound is particularly preferably 3-methyl-1-phenyl-pyrazolone (PMP).

A concentration of the cyclic active methylene compound in the Michael addition reaction in the substitution-modification step is not particularly limited, but as a concentration in the reaction solution, generally 0.05 mol/L to 1 mol/L, and preferably 0.1 mol/L to 0.5 mol/L.

As the cyclic active methylene compound, a compound represented by Formula (1) can be used. The cyclic active methylene compound may be used in combination of two or more types of compounds.

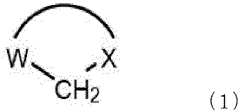

In Formula (1), W and X each represent a substituent containing an electron withdrawing group, and W and X are linked to each other to form a 5-membered ring or a 6-membered ring. Examples of the electron withdrawing group contained in W and X include a group having a carbonyl bond or an azomethine bond.

Representative examples of the active methylene compound include a pyrazolone compound represented by Formula (2), a barbituric acid compound represented by Formula (3), a dimedone compound represented by Formula (4), a hydroxycoumarin compound represented by Formula (5), and the like.

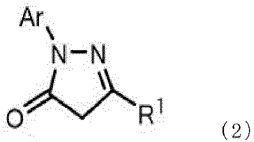

In Formula (2), Ar represents an aromatic ring which may have one or more substituents. Examples of the aromatic ring include a benzene ring, a pyrimidine ring, a pyridazine ring, a pyrazine ring, and a pyridine ring. Examples of the substituent of these aromatic rings include an alkyl group having 1 to 8 carbon atoms, an alkoxy group having 1 to 8 carbon atoms, a monoalkylamino group having 1 to 8 carbon atoms, a dialkylamino group having 2 to 16 carbon atoms, a halogen atom, and a cyano group. R¹ represents an alkyl group having 1 to 8 carbon atoms.

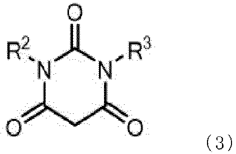

In Formula (3), R² and R³ each independently represent a hydrogen atom, an alkyl group having 1 to 8 carbon atoms, an alkoxycarbonylalkyl group having 3 to 16 carbon atoms, a carbamoylalkyl group having 2 to 9 carbon atoms, or an aromatic ring which may have a substituent. Examples of the aromatic ring include a benzene ring, a pyrimidine ring, a pyridazine ring, a pyrazine ring, a pyridine ring, a pyrrole ring, a pyrazole ring, an imidazole ring, a triazole ring, a tetrazole ring, and the like. Examples of the substituent of these aromatic rings include an alkyl group having 1 to 8 carbon atoms, an alkoxy group having 1 to 8 carbon atoms, a monoalkylamino group having 1 to 8 carbon atoms, a dialkylamino group having 2 to 16 carbon atoms, a halogen atom, a cyano group, and the like.

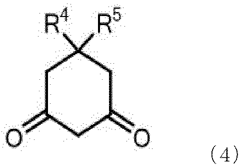

In Formula (4), R⁴ and R⁵ each independently represent an alkyl group having 1 to 4 carbon atoms.

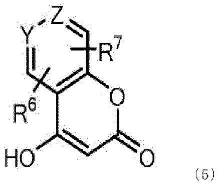

In Formula (5), Y and Z each independently represent -CH= or a nitrogen atom. R⁶ and R⁷ each independently represent a hydrogen atom, an alkyl group having 1 to 8 carbon atoms, an alkoxy group having 1 to 8 carbon atoms, a monoalkylamino group having 1 to 8 carbon atoms, a dialkylamino group having 2 to 16 carbon atoms, a halogen atom, or a cyano group.

Specific examples of the compounds represented by Formulae (2) to (5) are shown below.

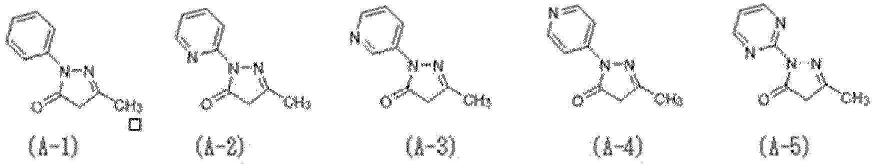

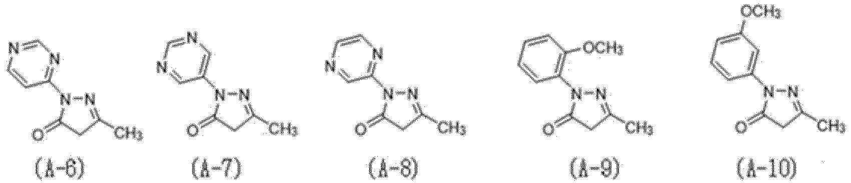

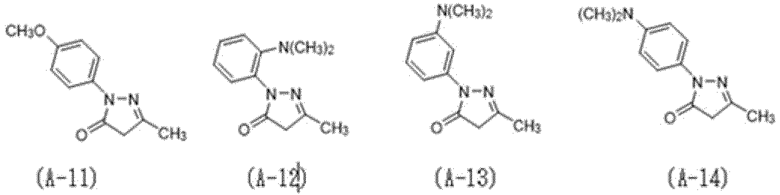

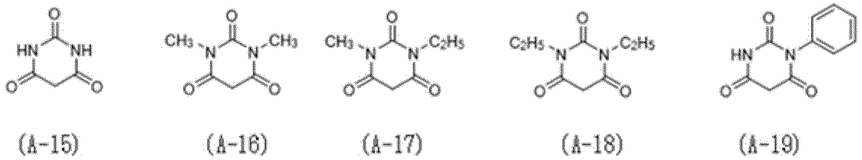

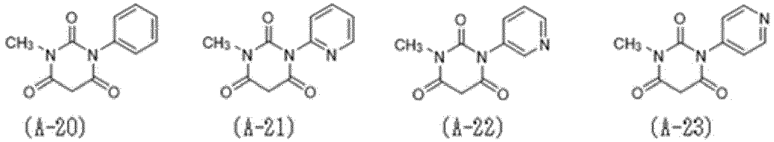

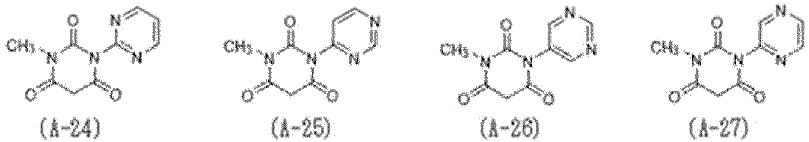

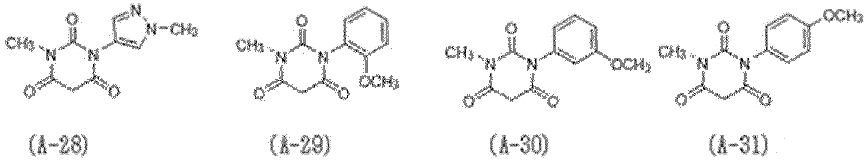

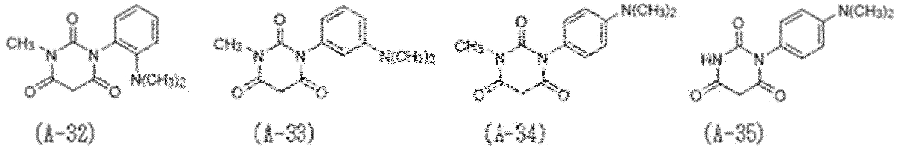

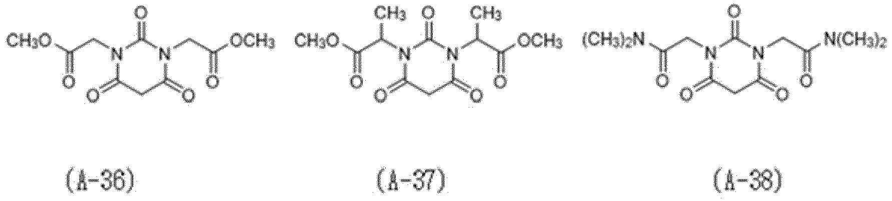

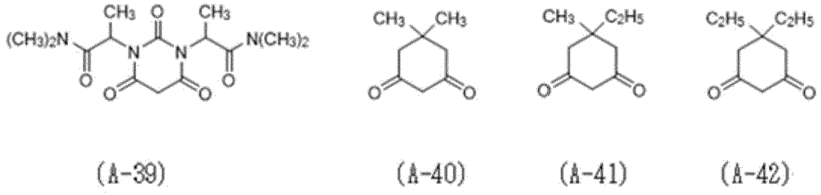

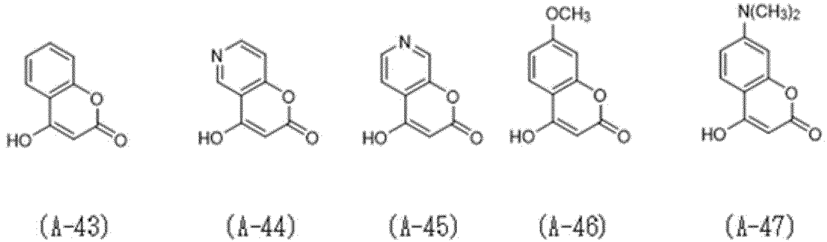

### <Fragmentation step of fragmenting protein>

The fragmentation step of fragmenting the protein can be performed by acting a proteolytic enzyme on the protein. As the pretreatment, the protein may be fragmented after the protein is reduced and carbamidomethylated. As the reduction of the protein, the cysteine residue in the protein can be reduced, for example, by acting a reducing agent such as dithiothreitol on the protein. Thereafter, the reduced protein can be carbamidomethylated by adding iodoacetamide thereto. Thereafter, the protein can be fragmented by acting the proteolytic enzyme on the protein. The enzyme that fragments the protein is preferably an enzyme that can cleave the protein between 5 residues or more and 100 residues or less, more preferably an enzyme that can cleave the protein between 6 residues or more and 60 residues or less, and particularly preferably an enzyme that can cleave the protein between 7 residues or more and 40 residues or less. Examples of the proteolytic enzyme include trypsin, chymotrypsin, pepsin, papain, ficin, bromelain, Lys-C (Promega Corporation), Asp-N (Promega Corporation), Arg-C (Promega Corporation), proteinase K, lysyl endopeptidase, V8 protease, and the like, and trypsin is preferable. In order to fragment into a preferred number of residues, it is also possible to fragment with a plurality of types of enzymes as appropriate. The amount of the proteolytic enzyme used is preferably 1/20 to 1/100 times the amount of the protein in the sample in terms of weight ratio.

In the present invention, the order of the substitution-modification step and the fragmentation step of fragmenting the protein is not particularly limited. That is, the fragmentation step of fragmenting the protein may be performed after the substitution-modification step is performed, or the substitution-modification step may be performed after the fragmentation step of fragmenting the protein is performed.

### <First mass spectrometry step and second mass spectrometry step>

The first mass spectrometry step in the present invention is a step of performing mass spectrometry on the fragmented protein, and is a step of confirming that the protein is glycosylated and further specifying a range of the glycosylated amino acid residues.

The second mass spectrometry step in the present invention is a step of analyzing a protein obtained by performing a substitution-modification step of substituting and/or modifying an amino acid at an amino acid site in the protein to which a sugar is bonded, and a fragmentation step of fragmenting the protein, for the protein in which the bonding of the sugar has been confirmed in the first mass spectrometry step.

A method of the mass spectrometry is not particularly limited, and as an example, the mass of an ion or a molecule can be measured by ionizing the molecule and measuring a mass-to-charge ratio (m/z) thereof.

### [Pretreatment]

In the first mass spectrometry, it is preferable to perform pretreatment of the sample before the mass spectrometry.

The pretreatment preferably includes reducing, carbamidomethylating, and fragmenting the protein. As the reduction of the protein, the cysteine residue in the protein can be reduced, for example, by acting a reducing agent such as dithiothreitol on the protein. Thereafter, the reduced protein can be carbamidomethylated by adding iodoacetamide thereto. Furthermore, the protein can be fragmented by acting the proteolytic enzyme on the protein. The enzyme that fragments the protein is preferably an enzyme that can cleave the protein between 5 residues or more and 100 residues or less, more preferably an enzyme that can cleave the protein between 6 residues or more and 60 residues or less, and particularly preferably an enzyme that can cleave the protein between 7 residues or more and 40 residues or less. Examples of the proteolytic enzyme include trypsin, chymotrypsin, pepsin, papain, ficin, bromelain, Lys-C, Asp-N, Arg-C, proteinase K, lysyl endopeptidase, and V8, and trypsin is preferable. In order to fragment into a preferred number of residues, it is also possible to fragment with a plurality of types of enzymes as appropriate. Furthermore, desalting of the fragmented protein (peptide) may be performed using GL-Tip SDB (GL Sciences Inc.) or the like as desired.

In the second mass spectrometry, as described above in the present specification, the mass spectrometry of the protein can be performed using a sample after performing the substitution-modification step and the fragmentation step (the order of the substitution-modification step and the fragmentation step is not a problem). Before the second mass spectrometry, desalting of the fragmented protein (peptide) may be performed using GL-Tip SDB (GL Sciences Inc.) or the like as desired.

### [Liquid chromatography/tandem type mass spectrometry]

The mass spectrometry of the pretreated sample can be performed using liquid chromatography (LC) and a mass spectrometer (MS). The liquid chromatography device and the mass spectrometer may be connected in series to each other, or only the mass spectrometer may be used. For example, an LC-MS system configured by connecting a liquid chromatography device and a mass spectrometer in series can be used. As the LC-MS system, a tandem type LC-MS/MS, LC-MS/MS/MS, or the like can be used.

### [Liquid chromatography device]

The liquid chromatography (LC) device is not particularly limited as long as it is a device capable of separating the glycosylated protein by liquid chromatography.

As the liquid chromatography, for example, high performance liquid chromatography (HPLC), ultra-high speed high separation liquid chromatography (UHPLC, UPLC, or UFLC), or low flow LC can be used, and an appropriate device can be selected according to the amount of a sample or the like. As the liquid chromatography (LC), low flow LC is preferable. Examples of the low flow LC include nano flow liquid chromatography (nano LC), capillary LC, and micro LC, and among above, nano LC is particularly preferable.

The LC device generally includes a separation column and a pump that feeds a separation solution to the separation column. The LC device may include elements other than those described above, for example, an autosampler, a heater, a detector that detects a separated component, and the like. Examples of the detector include a UV detector and a fluorescence detector. For example, the detector can be connected between the column and the ion source (ionization unit).

### [Mobile phase]

As conditions of the separation solution (mobile phase) used in the liquid chromatography, the separation solution can be used without particular limitation as long as it satisfies the condition of being a solvent applicable to a mass spectrometer. For example, water, formic acid, acetonitrile, or the like can be used.

### [Liquid chromatography conditions]

Conditions of the separation column used in the liquid chromatography are not particularly limited, and can be appropriately selected. As the separation column, a reverse phase column can be used. Examples of the reverse phase column include a column filled with an octadecylsilylated silica gel filler and a column in which an ion exchange resin is blended with these, and particularly, a column (ODS column) used in reverse phase chromatography, in which a filler is packed in which an octadecylsilyl group (ODS group, C18 group) is chemically bonded to a silica gel carrier, is preferable. For example, MonoCap C18 High Resolution 750 (GL Sciences Inc.) can be used as the separation column.

The gradient of the concentration of the mobile phase can be formed by mixing two or more types of solutions having different compositions while changing the mixing ratio. The combination of the solutions can be appropriately selected such that a desired gradient is formed.

The flow rate can be appropriately selected according to various conditions such as the inner diameter of the separation column. A flow rate of the separation solution may be constant throughout the separation step, or may not be constant. For example, the flow rate can be appropriately selected in a range of 100 nL/min to 100 µL/min.

A column temperature in the liquid chromatography can be appropriately selected by those skilled in the art. For example, the temperature is 20°C to 70°C, preferably 25°C to 50°C.

### [Mass spectrometry device]

The liquid chromatography/tandem type mass spectrometry fragment ion analysis method is a device consisting of a liquid chromatography unit and a mass spectrometry device unit, and further has a portion where the mass spectrometry unit can decompose and detect the precursor ion.

As the mass spectrometry device unit, a normal mass spectrometer can be used. The mass spectrometer may be one or two or more. Two or more mass spectrometers can be used by being connected in series. That is, the LC-MS system may be an LC-MS/MS or an LC-MS/MS/MS.

As the detection method of a tandem type mass spectrometer, a magnetic field deflecting type, a quadrupole type, an ion trap type, a time-of-flight type, an orbitrap type, or a hybrid type thereof can be used. A quadrupole/orbitrap type tandem mass spectrometer is preferable.

Examples of the ionization method in the mass spectrometer include an electrospray ionization (ESI) method, a matrix assisted laser desorption ionization (MALDI) method, an electron ionization (EI) method, a chemical ionization (CI) method, a field desorption (FD) method, a fast atom bombardment (FAB) method, an atmospheric pressure chemical ionization (APCI) method, an inductively coupled plasma (ICP) method, or the like. Among these, an ESI method is preferable.

The quadrupole/orbitrap type tandem mass spectrometer is a mass spectrometer composed of an ion source, a quadrupole (Q1), a collision cell (Q2), an orbitrap (Q3), and a detector. The measurement target is ionized by the ion source, the precursor ions are generated, and the precursor ions are separated by mass by the quadrupole (Q1) based on the mass-to-charge ratio (m/z) of the precursor ions. Next, the product ion is generated by colliding the separated precursor ions with an inert gas such as nitrogen or argon in the collision cell (Q2) (collision-induced dissociation: CID). The product ions are separated by mass again in the orbitrap (Q3) based on the mass-to-charge ratio (m/z), and are detected by the detector.

In the method according to the embodiment of the present invention, in the first mass spectrometry step, it is possible to clarify that the peptide is glycosylated from the spectrum of the mass-to-charge ratio (m/z) of the precursor ion of the peptide and the mass-to-charge ratio of the product ion of the peptide. In a range of amino acid residues of the peptide which has been found to be glycosylated, by the subsequent substitution-modification step, fragmentation step, and second mass spectrometry step, the amino acid residue (that is, the amino acid residue to be glycosylated) labeled from the spectrum of the mass-to-charge ratio (m/z) of the precursor ion of the peptide and the mass-to-charge ratio of the product ion of the peptide can be specified.

In addition, the modification rate of the sugar chain can be quantified based on the result of the mass spectrometry. The quantification of the modification rate of the sugar chain can be performed, for example, as follows. The modification rate is determined by quantifying the number of molecules of the measurement target peptide (unmodified peptide and GlcNAc-modified peptide, which have the same amino acid sequence) present in the sample. For the quantification, the measurement target peptide and stable isotopes preparation thereof are used. The stable isotope is used as an internal standard (IS) for the measurement. A calibration curve is acquired using a known amount of a peptide preparation to which an internal standard has been added, and each measurement target peptide in a sample is quantified using the calibration curve. The quantification based on the result of the mass spectrometry is determined from a ratio of a peak area obtained from an extracted chromatogram of a precursor ion corresponding to each measurement target peptide to a peak area obtained from an extracted chromatogram of a precursor ion corresponding to each IS. After quantifying the number of molecules of each measurement target in the sample, the modification rate can be quantified based on the following expression. Modification rate = amount of GlcNAc-modified peptide/(amount of unmodified peptide + amount of GlcNAc-modified peptide)

### <Kit>

According to the present invention, a kit for performing the method for identifying the amino acid site in the protein, to which the sugar is bonded according to the present invention, the kit containing a cyclic active methylene compound, is provided. Specific examples and preferred embodiments of the cyclic active methylene compound are as described above in the present specification.

The kit according to the embodiment of the present invention may further include a unit for purifying the fragmented protein. Examples of the unit for purifying the fragmented protein include solid phase extraction using hydrophobic interaction (a solid phase in which a filler having an ODS group chemically bonded thereto is packed).

The kit according to the embodiment of the present invention may include a protocol in writing for performing the method for identifying the amino acid site in the protein, to which the sugar is bonded according to the embodiment of the present invention. This protocol includes, for example, an identification procedure and information required for the identification.

The kit according to the embodiment of the present invention may further include a reagent used in the above-described method. Examples of the reagent include a proteolytic enzyme, a buffer, and the like.

The present invention will be described in more detail with reference to the following examples, but the present invention is not limited to the examples.

### Examples

### (1) Qualitative analysis of O-GlcNAc-modified peptide

### <Pretreatment>

90 µL of ice-cold ethanol was added to a 10 µL sample (285 fmol of AAV), and the mixture was allowed to stand at -20°C for 1 hour or more. After the centrifugation at 15,000 × g at 4°C for 10 minutes, the supernatant was removed. 100 µL of ice-cold ethanol was added to the AAV pellet, then the mixture was centrifuged at 15,000 × g at 4°C for 5 minutes to remove the supernatant, and the AAV pellet was air-dried to purify and inactivate the AAV

The AAV pellet was dissolved in 20 µL of MPEX PTS Reagent B (GL Sciences Inc.), and dithiothreitol (DTT) was added thereto such that a final concentration was 5 mmol/L, and the mixture was allowed to stand at room temperature for 30 minutes, thereby cysteine residues in the protein was reduced. Thereafter, iodoacetamide (IAA) was added thereto such that a final concentration was 25 mmol/L, and the mixture was allowed to stand at room temperature under light shielding for 30 minutes to carbamoylmethylate. The mixture was diluted 5 times with 77 µL of 50 mmol/L ammonium bicarbonate, 2 µL of 0.05 µg/µL trypsin was added thereto, and the mixture was allowed to stand at room temperature overnight to fragment the peptide of the AAV pellet. The sample was mixed with an equal amount (v/v) of ethyl acetate and a 1/100-fold amount (v/v) of trifluoroacetic acid (TFA) for 1 minute using a vortex mixer, and centrifuged at 15,600 × g at room temperature for 2 minutes, and the upper layer was removed. The sample was concentrated under reduced pressure at 30°C using a centrifugal evaporator CVE-3100 (TOKYO RIKAKIKAI CO., LTD.) until ethyl acetate in the sample was volatilized. 50 µL of a liquid A [5% (v/v) acetonitrile and 0.1% (v/v) trifluoroacetic acid] was added to the sample.

20 µL of a liquid B [80% (v/v) acetonitrile and 0.1% (v/v) trifluoroacetic acid] was put in GL-Tip SDB (GL Sciences Inc.), and passed through the SDB by centrifugation at 3,000 × g for 2 minutes. 20 µL of the liquid A was put therein, and passed through the SDB by centrifugation at 3,000 × g for 5 minutes, and then the sample was put therein, and passed through the SDB at 3,000 × g for 5 minutes by centrifugation to retain the peptide in the above-described SDB. 20 µL of the liquid A was put therein, and passed through the SDB by centrifugation at 3,000 × g for 5 minutes to wash the SDB. Then, 40 µL of the liquid B was put therein, and the peptide was eluted from SDB by centrifugation at 3,000 × g for 2 minutes. The solvent was removed by drying at 30°C using a centrifugal evaporator CVE-3100, and the residue was adjusted to 25 µL with a solution [2% (v/v) acetonitrile and 0.1% (v/v) formic acid].

### <NanoLC/MS/MS analysis and database search>

As the device, Ultimate 3000 Nano and Q Exactive (Thermo Fisher Scientific Inc.) were used.

As the trap column, L-column ODS having a diameter of 0.3 × 5 mm (particle diameter of 5 µm) (CERI) was used.

As the separation column, MonoCap C18 High Resolution 750 (GL Sciences Inc.) was used.

The flow rate was 500 nL/min. As the mobile phase A, 0.1% formic acid (in H₂O) was used, and as the mobile phase B, acetonitrile was used. The gradient conditions were set to %B = 4% (0 min) → 45% (80 min) → 80% (85 min) → 80% (95 min) → 4% (96 min) → 4% (120 min). A column temperature was set to 35°C, and an injection volume was 10 µL.

Detection was performed using ESI-MS (positive mode), and a capillary voltage was set to 2 kV. The MS scan range was set to m/z 350 to 1,800 (resolution of 70,000, AGC of 3 × 10⁶), and the top 10 precursor ions in each MS scan were fragmented by HCD and then subjected to MS/MS scan (resolution of 35,000, AGC of 1 × 10⁵). The standard collision energy was set to 25%, and the dynamic exclusion time was set to 30 seconds. An isolation width was set to 2.0 m/z.

Database search was used for screening of the peptide qualitative analysis. For the database analysis, Proteome Discoverer Ver1.3 (Thermo Fisher Scientific Inc.) was used, and Sequest HT (Thermo Fisher Scientific Inc.) and Mascot (Matrix Science Ltd.) were used as a database search engine. The allowable number of uncut trypsin was set to 2 or less, the allowable precursor mass error was set to 10 ppm, the fragment mass error was set to 20 mmu, the fixed modification was set to carbamidomethylation (C), and the dynamic modification was set to oxidation (O) and HexNAc (S and T). As a database, AAV9 (Q6JC40) (UniProt registration number Q6JC40) was used. The false identification rate was calculated based on a decoy database (reverse amino acid sequence of the database), and was set to less than 1% at the peptide level. The MS/MS spectrum of each peptide hit by the search was manually checked, and a peptide having a mass error of 10 ppm or less with respect to the theoretical value of the fragment ion was adopted as the basis of the qualitative analysis. Among the glycopeptide candidates hit by the search, glycopeptide candidates in which a sugar-derived ion group (m/z 204 and two or more other fragment ions) was observed in the MS/MS spectrum, were determined as a glycopeptide.

### <Result>

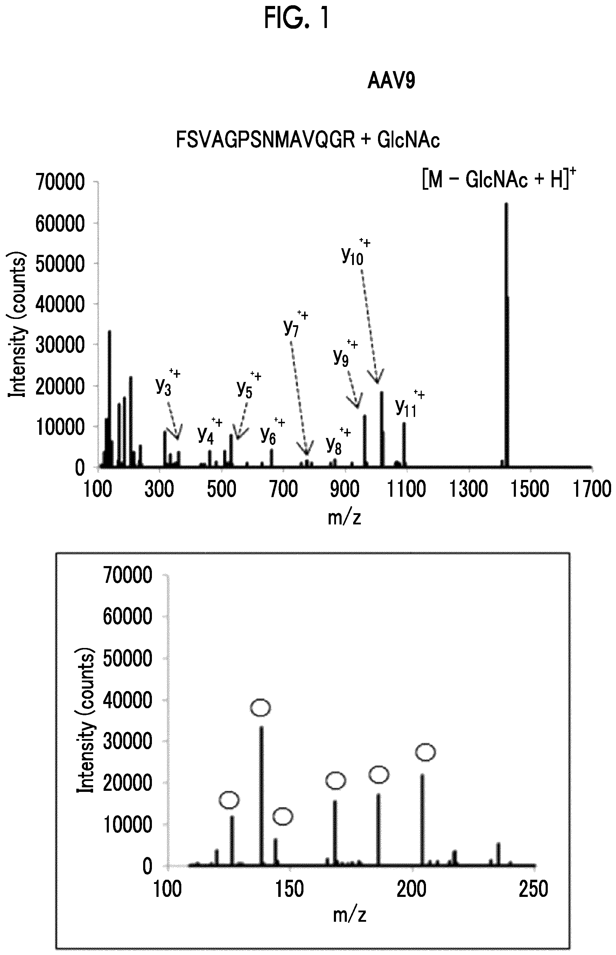

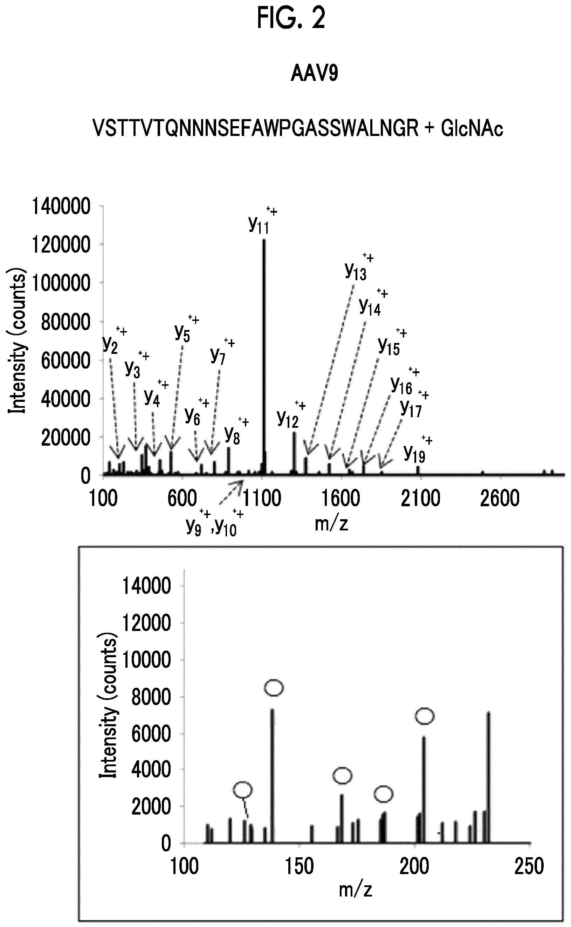

The MS/MS spectra of the O-GlcNAc-bonded peptide detected from AAV are shown in Figs. 1 and 2.

The FSVAGPSNMAVQGR shown in Fig. 1 shows the amino acid sequence at the amino acid positions 463 to 476 of the AAV.

The VSTTVTQNNNSEFAWPGASSWALNGR shown in Fig. 2 shows the amino acid sequence of the amino acid positions 489 to 514 of the AAV.

From the results of Fig. 1, it was found that one O-GlcNAc was bonded to the amino acid positions 463 to 476 of AAV9. From the results of Fig. 2, it was found that one O-GlcNAc was bonded to the amino acid positions 489 to 514 of AAV9.

In the upper left figures of Fig. 1 and Fig. 2, the peaks indicated by a circle represent oxonium ions derived from GlcNAc.

The y ion series in Fig. 1 and Fig. 2 (ys to Yn in Fig. 1 and y₂ to y₁₇ and y₁₉ in Fig. 2) indicate peaks derived from a peptide from which GlcNAc is removed.

As shown in Fig. 1 and Fig. 2, in a case where the O-GlcNAc-bonded peptide is directly detected, since the GlcNAc is removed during fragmentation, the bonding site information of the GlcNAc is not left on the MS/MS spectrum. Therefore, in a case where a plurality of Ser or Thr residues are present in a peptide, a site of GlcNAc cannot be uniquely specified. Thus, a method of substituting GlcNAc bonded to a peptide with a stable substituent with respect to fragmentation and leaving bonding site information on the MS/MS spectrum was performed.

### (2) Estimation of modification site of O-GlcNAc-modified peptide

### <Pretreatment>

The AAV was purified and inactivated in the same manner as in (1) described above using ice-cold ethanol, and the AAV pellet was air-dried. 10 µL of water, 20 µL of 0.4 mol/L NaOH, and 20 µL of 0.5 mol/L 1-phenyl-3-methyl-5-pyrazolone (PMP) were added to the air-dried AAV pellet, and the mixture was allowed to stand at 85°C for 16 hours (At this time, β-elimination of the sugar from the glycopeptide and Michael addition of PMP to the peptide were performed). 450 µL of ice-cold ethanol was added thereto, and the mixture was allowed to stand at -20°C for 1 hour or more. After the centrifugation at 15,000 × g at 4°C for 10 minutes, the supernatant was removed. The AAV pellet was washed twice with 500 µL of ice-cold ethanol. The centrifugation during the washing was performed at 15,000 × g and 4°C for 5 minutes. The dried AAV pellet was dissolved in 20 µL of MPEX PTS Reagent B, and reduction with DTT, alkylation with IAA, and fragmentation with trypsin digestion (fragmentation step) were performed in the same manner as in (1) described above. The desalting of the peptide was also performed in the same manner as in (1) described above using GL-Tip SDB.

### <NanoLC/MS/MS analysis and database search>

Unless otherwise specified, the same conditions as in (1) described above were adopted. As the MS conditions, MS was performed at the same conditions as in (1) described above. Dynamic modification of the database search was set to oxidation (O), deamidation (N), and PMP modification (S and T, change in mass of +156.06875). The MS/MS spectrum of each peptide hit by the search was manually checked, and a peptide having a mass error of 10 ppm or less with respect to the theoretical value of the fragment ion was adopted as the basis of the qualitative analysis. The PMP modification is a modification of an amino acid residue with PMP.

### <Result>

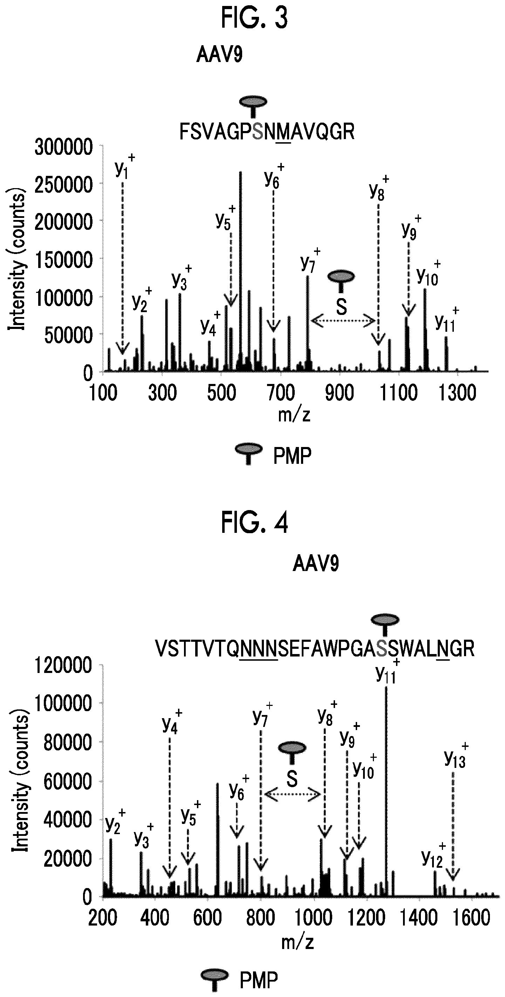

MS/MS spectra of the PMP-modified, AAV-derived peptide are shown in Fig. 3 and Fig. 4.

As shown in Fig. 3, since the difference in mass-to-charge ratio between the y₈⁺ ion and the y₇⁺ ion of the peptide precisely matched the mass of serine to which PMP was added, it was found that the 8th serine from the carboxy terminal of the peptide (S469 of AAV9) was PMP-modified. As shown in Fig. 4, since the difference in mass-to-charge ratio between the y₈⁺ ion and the y₇⁺ ion of the peptide precisely matched the mass of serine to which PMP was added, it was found that the 8th serine from the carboxy terminal of the peptide (S507 of AAV9) was PMP-modified. That is, it was detected that O-GlcNAc was bonded to S469 and S507 of AAV9.

### (3) Quantification

### <Pretreatment>

90 µL of ice-cold ethanol was added to a 10 µL sample (285 fmol of AAV), and the mixture was allowed to stand at -20°C for 1 hour or more. After the centrifugation at 15,000 × g at 4°C for 10 minutes, the supernatant was removed. 100 µL of ice-cold ethanol was added to the AAV pellet, and then the mixture was centrifuged at 15,000 × g at 4°C for 5 minutes to remove the supernatant, and the AAV pellet was air-dried. 17 µL of MPEX PTS Reagent B and 3 µL of a solution of the measurement target peptide labeled with a stable isotope (10 ppm [¹³C₅,⁵N₅]FSVAGPSNMAVQGR, 10 ppb [¹³C₅,⁵N₅]FSVAGP(GlcNAc-S)NMAVQGR) were added to the AAV pellet, and reduction, alkylation, and trypsin digestion were performed in the same manner as in (1) described above. Desalting of the peptide was also performed in the same manner as in (1) described above.

### <NanoLC/MS/MS analysis and analytical study>

The same conditions as in (1) and (2) described above were adopted.

The ion extraction conditions used for the peptide quantification are as follows.
FSVAGPSNMAVQGR (m/z of 710.8559)
FSVAGP(GlcNAc-S)NMAVQGR (m/z of 715.8578)
[¹³C₅,⁵N₅]FSVAGPSNMAVQGR (m/z of 812.3959)
[¹³C₅,⁵N₅]FSVAGP(GlcNAc-S)NMAVQGR (m/z of 817.3976)

### <Result>

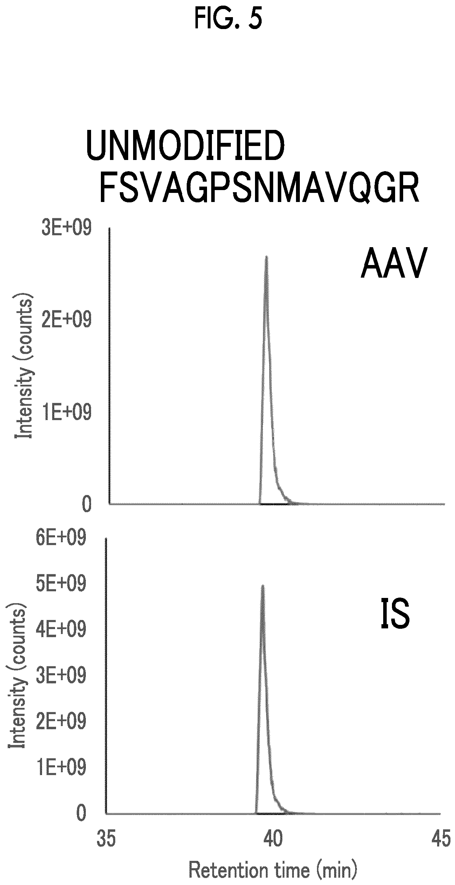

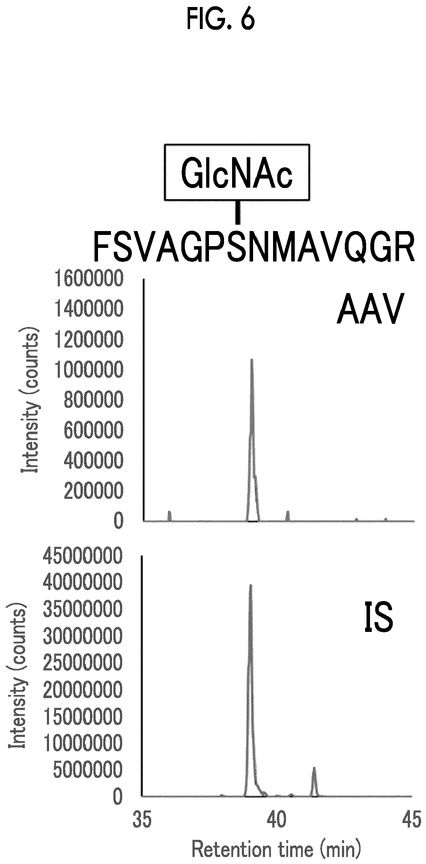

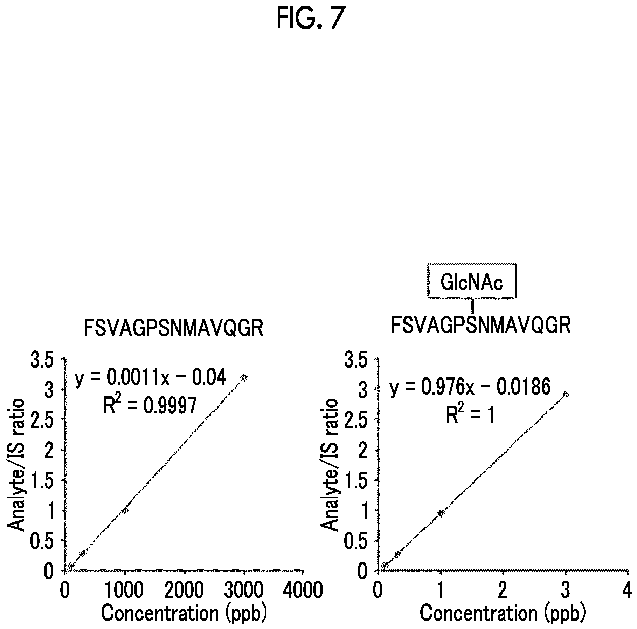

Fig. 5 shows an extracted chromatogram of a precursor ion for an unmodified peptide preparation and an internal standard (IS). Fig. 6 shows an extracted chromatogram of a precursor ion for a peptide preparation in which S469 is modified and the internal standard (IS). The peak surface area of each peptide was calculated from the extracted chromatogram of the precursor ion corresponding to each peptide. The quantification of each peptide was performed based on a peak surface area ratio of the measurement target peptide (unmodified and GlcNAc-modified peptide) and the IS corresponding thereto. Each peptide preparation to which IS was added was used to create the calibration curve. The obtained calibration curve is shown in Fig. 7.

The modification rate was calculated in terms of molar based on modification rate = number of GlcNAc-modified peptide molecules/(number of unmodified peptide molecules + number of GlcNAc-modified peptide molecules).

The modification rate was 0.4%.

### [Sequence list]

International application 21F01566W1JP23000038_9.xml based on International Patent Cooperation Treaty

## Claims

1. A method for identifying an amino acid site in a protein to which a sugar is bonded, the method comprising:
a first mass spectrometry step of subjecting a fragmented protein to mass spectrometry;
for the protein in which the bonding of the sugar has been confirmed in the first mass spectrometry step, a substitution-modification step of substituting and/or modifying an amino acid at the amino acid site in the protein to which the sugar is bonded and a fragmentation step of fragmenting the protein; and
a second mass spectrometry step of subjecting the substituted and/or modified, and fragmented protein obtained by the substitution-modification step and the fragmentation step, to mass spectrometry.

2. The method according to claim 1, further comprising:
comparing a mass spectrometry result obtained in the first mass spectrometry step with a mass spectrometry result obtained in the second mass spectrometry step.

3. The method according to claim 1 or 2,
wherein the amino acid to which the sugar is bonded is serine and/or threonine.

4. The method according to any one of claims 1 to 3,
wherein the substitution-modification step is a substitution reaction by a Michael addition reaction to an amino acid site from which the sugar has been eliminated by a β-elimination reaction.

5. The method according to any one of claims 1 to 4,
wherein the Michael addition reaction in the substitution-modification step is a reaction with a cyclic active methylene compound.

6. The method according to any one of claims 1 to 5,
wherein the Michael addition reaction in the substitution-modification step is a reaction with a pyrazolone compound, a barbituric acid compound, a dimedone compound, or a hydroxycoumarin compound.

7. The method according to any one of claims 1 to 6,
wherein the Michael addition reaction in the substitution-modification step is a reaction with a pyrazolone compound.

8. The method according to claim 7,
wherein the pyrazolone compound is 3-methyl-1-phenyl-pyrazolone.

9. The method according to any one of claims 1 to 8,
wherein the sugar is N-acetylglucosamine.

10. The method according to any one of claims 1 to 9,
wherein the protein is a capsid of a virus.

11. The method according to claim 10,
wherein the virus is an adeno-associated virus.

12. The method according to claim 11,
wherein the virus is an adeno-associated virus 5 referred to as AAV5.

13. The method according to any one of claims 1 to 12,
wherein an amount of the protein to be subjected to the spectrometry is 100 fmol or less.

14. A kit for performing the method according to any one of claims 1 to 13, the kit comprising:
a cyclic active methylene compound.

15. The kit according to claim 14, further comprising:
a unit for purifying a fragmented protein.
